# EUROPEAN PATENT SPECIFICATION

(11) **EP 2 375 970 B1**
(45) Date of publication and mention of the grant of the patent: **03.12.2014**
(21) Application number: 08876223.2
(22) Date of filing: 29.10.2008
(51) Int. Cl.: A61B 5/022

(54) **A BLOOD PRESSURE MEASUREMENT DEVICE AND A FRONT END**
BLUTDRUCKMESSGERÄT
DISPOSITIF DE MESURE DE PRESSION SANGUINE ET EXTRÉMITÉ ANTÉRIEURE

(43) Date of publication of application: 19.10.2011
(73) Proprietor: Bmeye B.V., 1105 AZ Amsterdam (NL)
(72) Inventor: GUELEN, Ilja, NL-5371 DJ Ravenstein (NL); VAN DER SAR, Geertruida, Lucretia, NL-2404 VJ Alphen aan den Rijn (NL); SCHRAA, Bob, NL-1191 NG Amsterdam (NL)
(74) Representative: Jansen, Cornelis Marinus
(86) International application number: PCT/NL2008/050677
(87) International publication number: WO 2010/050798

(56) References cited:
- EP-A- 0 399 189
- WO-A-2005/037097
- US-A- 4 406 289
- US-A- 4 862 895
- US-A- 5 662 092
- US-B1- 6 171 254

## Description

### FIELD OF THE INVENTION

The invention relates to a blood pressure measurement device. More particularly, the invention relates to a non-invasive cuff-based blood pressure measurement device. The invention further relates to a front end for use in a blood pressure measurement device.

### BACKGROUND OF THE INVENTION

A blood pressure measurement device as is set forth in the foregoing is known in the art. The known blood pressure measurement device may comprise an inflatable cuff arranged to induce pressure in a tissue for causing said tissue to substantially tightly enclose a suitable blood vessel running in said tissue for purposes of blood pressure measurement. An embodiment of a cuff-based non-invasive blood pressure measuring device is known from US 4, 726, 382. The known cuff comprises an inflatable bladder formed from a thin flexible, translucent material. The inflatable bladder is connected to a tube enabling a suitable inflation and deflation of the inflatable bladder. The inflation and deflation of the inflatable bladder is controlled by a control valve, which may be operated pneumatically or electrically. In the known cuff the inflatable bladder is arranged as an innermost component of the cuff. The inflatable bladder may be manufactured from two strips of film being heat sealed together about their periphery thereby forming a cavity. The known cuff may be arranged to be fit about a person's finger. In order to implement a suitable collection of a signal or data representative of a blood pressure measurement, the known cuff comprises a photoplethysmograph.

It is a disadvantage of the known blood pressure measuring device that the valve controlling inflation and deflation of the inflatable body may disadvantageously affect gas flow patterns in the device causing deterioration of accuracy of the blood pressure measurement.

It is noted that patent publication WO 2005/037097 discloses a system for measuring blood pressure, wherein use may be made of a cuff. The system includes an air chamber that is in direct fluidal connection with the cuff. It is further noted that patent publication US 5 662 092 discloses an appliance for measuring blood pressure wherein an air chamber is provided between a cuff and an air tank pressurized by air pumping means. It is also noted that patent publication US 4 862 895 discloses an electronic blood pressure meter which is adapted to measure blood pressure from a finger. The blood pressure meter includes an air buffer.

### SUMMARY OF THE INVENTION

It is an object of the invention to provide a blood pressure measurement device with improved operational characteristics. More particularly, it is an object of the invention to provide a blood pressure measurement device having improved accuracy.

To this end a front end of the blood pressure measurement device according to the invention is defined in claim 1.

It is found to be advantageous to provide a buffer gas volume upstream the control valve, for example directly prior to the control valve in the gas conduit between the control valve and a suitable gas supply unit. This feature is based on the following insights. For situations when pressure in the first conduit is to be rapidly increased for rapidly increasing pressure in the inflatable body, the control valve has to be set open so that an input pressure from the gas supply unit may substantially be fully applied to the inflatable body. Such arrangement may cause a substantial distortion of flow patterns in the second portion of the gas conduit leading to a temporary reduction in pressure in the second portion of the gas conduit. In order to prevent such distortion of flow patterns, the buffer gas volume, preferably provided directly prior to the control valve in the second portion of the gas conduit, is provided. It is found that due to provision of the buffer gas volume the measurement accuracy of the blood pressure measurement device substantially improves. Preferably, the system for measuring a signal representative of the blood pressured comprises a photoplethysmograph.

In an embodiment of the blood pressure measurement device according to the invention, the frontend is accommodated in a housing, the air chamber forming a part of the housing. Due to this feature the number of constructive parts of the frontend may substantially be reduced leading to a better cost-efficiency of the manufacturing process of the frontend. The housing is substantially made of plastic, which is injection molded. Preferably, the housing comprises or consists of a cover portion and a bottom portion.

In a further embodiment of the blood pressure measurement device, the control system is arranged for analyzing a signal representative of pressure measured by the pressure sensor or photoplethysmograph and for generating a control signal to the control valve in response to said signal.

The pressure sensor may form part of a control system of the blood pressure measurement device according to the invention. For instance, the pressure valve may form part of a so-called pressure loop. During the pressure loop the pressure in the inflatable body may be controlled with the control valve to realize a pre-determined pressure ("pressure setpoint") inside the inflatable body. Such pre-determined pressure may be pre-set by a processor of the device. Such setting may result in stable pressures inside the inflatable device during one or more heartbeats. It will be appreciated that advantageously the inflatable body is arranged to maintain a substantially constant diameter of a blood vessel during a blood pressure measurement. It will be further appreciated that the term 'blood vessel' may relate to any blood conduit inside a body, in particular to a peripheral blood conduit. During the stable pressure, a signal representative of blood pressure is picked-up by the system for measuring blood pressure. For example, such signal may relate to an electrical signal, derived from a suitable photoplethysmograph, which may be arranged in the inflatable body. This signal may be then analyzed, for example, by the processor, to derive a further suitable operational parameter, for example a so-called "volume clamp setpoint" to be used in a volume clamp loop. The volume clamp loop will be discussed below. In case when the actual pressure in the inflatable body, as being measured by the pressure sensor, is lower than the setpoint pressure then the control valve may be operated by the control system to increase the pressure in the inflatable body and vice versa.

In a still further preferred embodiment of the blood pressure measuring device according to the invention, the control system is arranged for analyzing a further signal from the system for measuring a signal representative of blood pressure and for generating a further control signal to the control valve in response to said further signal.

This embodiment relates to the volume clamp loop which represents an actual blood pressure measurement. During the volume clamp loop the pressure in the inflatable body may also be controlled with the control valve, but in this case the control system is arranged to be responsive to the electrical (or light) signal and not necessarily to the pressure sensor. The object of this control system is to maintain the measured electrical (or light) signal stable at the pre-determined volume clamp setpoint by rapidly changing the pressure inside the inflatable body. As a result the pressure inside the inflatable body accurately mimics the sought blood pressure. By measuring the pressure inside the inflatable body, using the pressure sensor, the blood pressure is determined. If the actual received signal (or light) is lower than the volume clamp setpoint then the control valve is operated to increase the pressure in the inflatable body. This results in an increase in the received light in the photoplethysmograph and vice versa.

The operation of the valve in both control loops may be similar, for example use of a 3-connection 2-way valve may be envisaged. Connection 1 may be connected to the air supply (pump via the air chamber), connection 2 may be connected to a lower pressure like the ambient surroundings or vacuum and connection 3 may be connected to the inflatable body. Using connections 1 and 2 the valve can be controlled to inflate and deflate the inflatable body.

It is possible to use a single control valve with 3 connections, however it is also possible to replace this control valve by two control valves with, for example, 2 connections, wherein one valve is responsible for connection between the supply and the inflatable body (for inflating it) and the second valve is responsible for the connection between the inflatable body and ambient (deflating the inflatable body).

Preferably, the device according to the invention further comprises a processor for collecting a signal or data representative of the blood pressure measurement. In particular, the device according to the invention may further comprise a monitor for receiving and/or for storing said signal or data.

In a still further embodiment of the device according to the invention, the device comprises a plurality of differently sized inflatable bodies.

It is found to be particularly advantageous to supply the device with a plurality of differently sized inflatable bodies. For example, a plurality of differently sized finger cuffs may be provided. The sizes may preferably vary in a range of 0.1 - 3 cm³. The size may range for application for a neonatal, an average adult, an obese adult, a male, a female, an aged person and so on. In this case the device advantageously may comprise an identifying unit for identifying a size of an inflatable body being connected to the device. This feature enables not only a fully automated control of a proper connection of a suitable inflatable body, but may suitably be used for further improving blood pressure measurement technique.

In a still further preferred embodiment of the blood pressure measuring device according to the invention, it comprises a control computer program arranged to adapt a control algorithm of the control valve in accordance with the size of the inflatable body and to cause the control system to carry-out the adapted control algorithm, the identifying unit providing input data to the control program.

As has been described above, the processor may determine (based on the measured pressure in pressure loop and based on the measured signal in volume clamp loop) whether the pressure inside the inflatable body has to increase, decrease or to remain unchanged. To accomplish this, the control system may be implemented in the software conceived to be run on the processor. For this purpose a control algorithm comprising suitable implementations and/or controllers may be chosen. For example, a PID (Proportional Integral Differential) controller, a PI controller, a Feedforward controller or a State Space controller. These controllers are per se known to the person skilled in the art and will not be explained in detail. In a suitable embodiment of the device according to the invention a PID controller may be selected for the volume clamp loop and a PI controller may be selected for the pressure loop. It has been found that this combination provides reliable blood pressure measurement results.

In accordance with the present embodiment, suitable parameters of the chosen control algorithms may be adapted for carrying out a particular measurement, for instance in dependence to the size of the inflatable body or the occurrence of oscillations or in dependence to analysis of the error signal (light signal during blood pressure measurement). For example, different gain factors of the PID controller, for example, the proportional gain, the integrating gain and the differentiating gain or the overall control gain may be adapted by the processor based on the aforementioned inputs. Preferably, the adaptation scheme is pre-stored in an automatically accessible look-up table. Alternatively, the adaptation scheme or the value to be used may be computed or determined on-line using a suitable algorithm or a pre-programmed logic.

Further advantageous embodiments of the blood pressure measurement device are set forth in the appended claims. These and other advantages of the blood pressure measurement device are further discussed with reference to drawings, wherein like reference numerals represent like items. It will be appreciated that the figures are used for illustrative purposes and may not be used for limiting the scope of the appended claims.

### BRIEF DESCRIPTION OF THE DRAWINGS

Figure 1 presents a block-scheme of an embodiment of the blood pressure measurement device according to the invention.
Figure 2 presents a schematic view of the housing of the frontend of the blood pressure measurement device according to the invention.
Figure 3a presents an elevated view of the housing of Figure 2.
Figure 3b presents a further portion of the elevated view of the housing of Figure 2.
Figure 3c presents a still further portion of the elevated view of the housing of Figure 2.
Figure 4 presents a schematic view of a gas flow pattern inside the housing shown in Figure 2.

### DETAILED DESCRIPTION

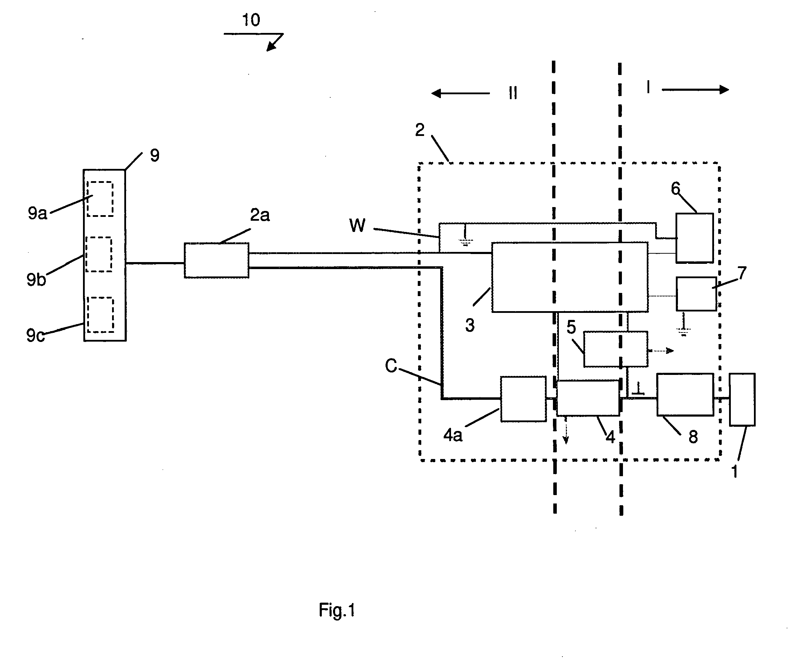

Figure 1 presents a block-scheme of an embodiment of a blood pressure measurement device according to the invention. The blood pressure measurement device 10 may comprise a monitor 9 connectable to a frontend unit 2, which in turn may be connectable to an inflatable body 1, for example a finger cuff. The frontend 2 may comprise necessary electrical wiring W and a gas conduit C necessary for functioning of the device 10. The frontend may preferably be arranged to perform an actual blood pressure measurement. To this end it may comprise all hardware and software that are needed for enabling such measurement. Preferably, suitable power supply 9a and gas supply 9b are located in the monitor 9. The monitor 9 may in addition be used to provide a trigger signal to the frontend 2 to start/stop a blood pressure measurement and to receive measurement signal and/or measurement data back from the frontend. More preferably, the frontend 2 may comprise suitable software for controlling a measurement mode as well as suitable program for carrying out preliminary data analysis of the blood pressure measurement data. For this purpose the frontend may comprise a processor (not shown) which may be arranged to control of the blood pressure measurement, as is described in the foregoing.

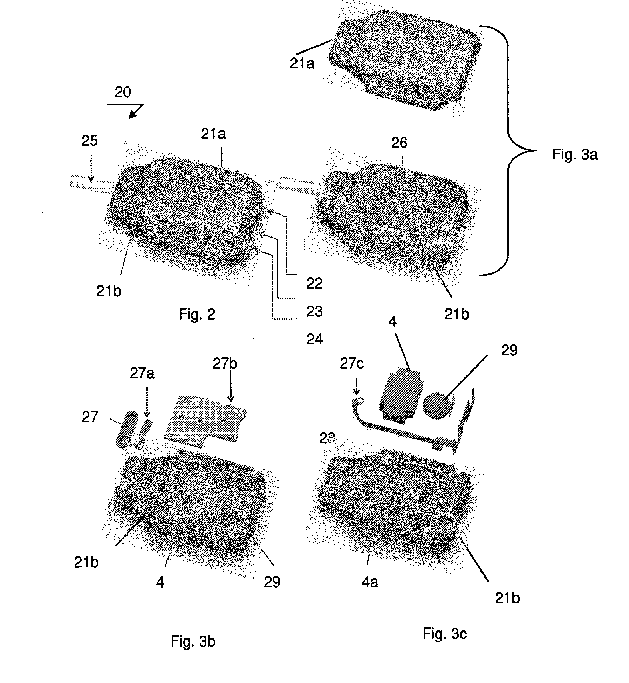

The frontend 2 may comprise the following functional units:
- a custom printed circuit board (PCB) 3, which may be built based on an off-the-shelf processor. Electronics on the PCB may provide interfaces between the processor, the cuff 1, the monitor 9 and other frontend components, like the control valve 4 and the pressure sensor 5;
- housing (shown in detail in Figures 2, 3a - 3c). All components of the frontend 2 are mounted to the housing, whereas the housing may be arranged to provide an electromagnetic shielding for the electronic components inside the housing;

- a pressure sensor 5, which may comprise off-the-shelf pneumatic control valve 4 that may be arranged to transform an electronic signal from a suitable processor into a desired pressure inside the inflatable body 1;
- a pressure transducer (not shown) that converts pressure readings as measured by the pressure sensor 5 into an electrical signal and to feed this signal to the processor.

The frontend 2 may further comprise a gas connector 8 to the inflatable body 1. Preferably the gas connector is connected by means of a suitable hose. Electrical connectivity of the printed circuit board to the inflatable body 1 may be enabled by means of a suitable, preferably, dedicated connector 6. This has an advantage that an automatic detection of an electrically connected inflatable body 1 may be enabled by the frontend 2. The printed circuit board may be connected via connector 7 to a heart reference system (HRS) used to provide a pressure reference height or connected to a pulse oximeter. A suitable gas to inflate or deflate the inflatable body 1 is provided from the gas source 9b located in the monitor 9 by means of a gas conduit C. The gas conduit C comprises a first portion I between the control valve 4 and the inflatable body and a second portion II, between the monitor 9 and the control valve 4.

In accordance with the invention, the blood pressure measuring device 10 is provided with a frontend 2 wherein a gas chamber 4a is provided in the second portion II of the gas conduit C, directly before the control valve 4 with respect to a direction of the stream towards the inflatable body 1. Due to this feature the pressure does not reduce dramatically in the second portion II just before the control valve for cases when the control valve 4 is fully open to pump gas in the first portion I of the gas conduit C. As a result the accuracy of the blood pressure measurement device is increased, because there is substantially no temporal pressure loss in the conduit.

The blood pressure measuring device 10 may be used, for example to measure arterial blood pressure and/or cardiac output and/or other cardiovascular parameters. Preferably, a finger cuff is used for this purpose. A start of a finger arterial pressure measurement may be initiated by a command sent by the monitor 9 to the frontend 2. Upon start, the frontend 2 enables due measurement conditions in terms of pressure inside the finger cuff and measures pressure data using the measurement system, for example based on photoplethysmograph. It is noted that operation of such measurement systems is known per se. In addition, the frontend 2 may initiate calculations of suitable physiologic parameters based on a signal or data provided by the measurement system of the finger cuff. The frontend 2 may further perform suitable actions needed for a proper measurement, like carrying out the pressure loop and/or the volume clamp loop as is described in the foregoing. In addition, the frontend 2 may further be arranged to detect heartbeats in the measured pressure waveform and derive from each beat either of the following physiological parameters:
- systolic, diastolic and mean finger arterial pressure;
- time of occurrence of the systolic and diastolic pressure;
- pulse rate and interbeat interval.

These beat data may be used internally in the frontend 2 or in the monitor software 9c to time periods for which suitable physiological data analysis is performed. In addition, these beat data may be used to check the blood pressure measurement.

In case when the frontend 2 is provided with a per se known heart reference system (HRS) connected to connector 7, the frontend may be able to correct for possible orthostatic pressure differences originating from a difference in height between the finger and the heart. To do so, a part of the HRS is located near to the finger cuff and another part is connected at heart level. Height correction by the frontend takes place by a combination of hardware outside the frontend (the HRS), and hardware and software inside the frontend. This embedded hardware and software is inactive in case no HRS is connected.

In addition, the frontend 2 may comprise an identification unit arranged to automatically determine a size of the inflatable body 1 connected to the frontend 2. The identification unit may be part of the photoplethysmograph but may also be part of the gas connection. Preferably, the inflatable body 1 comprises mechanic, pneumatic or electronic means for signalling its size.

Figures 2, 3a-3c present schematically structural build-up of structural components of the frontend 2. Figure 2 presents a schematic view of the housing of the frontend of the blood pressure measurement device according to the invention. The frontend 20 comprise an upper portion of the housing 21a and a lower portion of the housing 21b. Preferably, for ease of manufacturing, the housing 20 is injection molded. Figure 3a presents an elevated view of the housing of Figure 2. Inside the housing 21a and 21b a printed circuit board (PCB) 26 is positioned. The PCB may be arranged to provide electrical connectivity from the monitor (not shown) to all components of the frontend 2 and further to the inflatable body (not shown). Figure 3b presents a further portion of the elevated view of the housing of Figure 2 comprising a press-on plate 27b, the control valve 4 and pressure transducer 29. Further structural components of the frontend comprise a strain relief clamp 27 and a shielding clamp 27a. Figure 3c presents a still further portion of the elevated view of the housing of Figure 2. Below the press-on plate 27b a gas chamber 4a is shown which is arranged for providing a buffer gas volume to the control valve 4. The frontend further comprises a conducting brace 27c for providing suitable electrical contact between the external connectors (not shown) and the PCB.

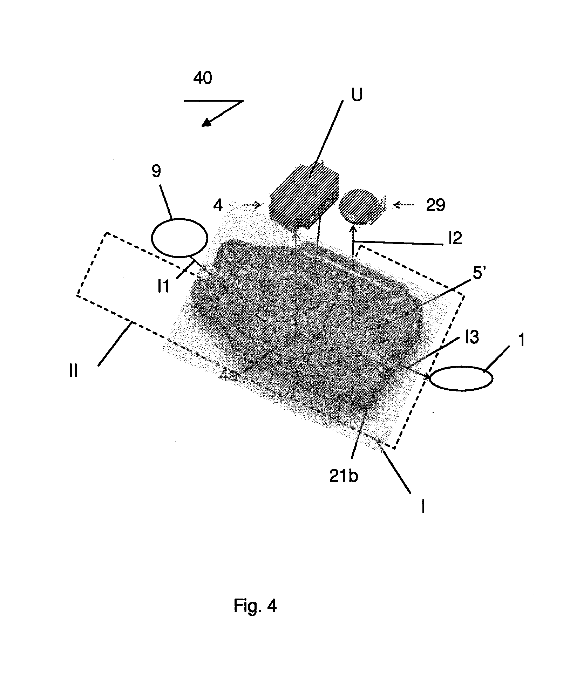

Figure 4 presents a schematic view of a gas flow pattern inside the housing shown in Figure 2. For the purpose of simplicity only the bottom portion 21b of the housing of the frontend 40 is shown. The bottom portion of the housing 21b is preferably injection moulded, wherein suitable cavities for the gas chamber 4a, for control valve 4 and for pressure sensor 5' are co-moulded. More preferably, a part of the first portion of the gas conduit I traversing the inner volume of the frontend 40 is co-molded as well. By provision of a direct and rigid, not interrupted gas conduit without passages between the gas chamber 4a, control valve 4 and pressure sensor 29 accuracy and reliability of the pressure measurement are increased.

Gas, preferably, ambient air, has a following flowing pattern inside the frontend 40. The flow pattern will be discussed with reference to inflating the inflatable body 1 from the gas supply 9. In this case, the incoming flow I1 traverses the second portion II of the gas conduit between the gas supply unit 9 and the control valve 4. The cavity 5' for the pressure sensor 29 is provided with an opening via which a stream of gas I2 escapes from the main stream I heading to the inflatable body 1. Preferably, the path I3 between the frontend 40 and the inflatable body 1 is provided in a suitable hose (not shown).

It will be appreciated that although specific embodiments of the blood pressure measuring device according to the invention are discussed separately for clarity purposes, interchangeability of compatible features discussed with reference to isolated figures is envisaged. While specific embodiments have been described above, it will be appreciated that the invention may be practiced otherwise than as described. The descriptions above are intended to be illustrative, not limiting. Thus, it will be apparent to one skilled in the art that modifications may be made to the invention as described in the foregoing without departing from the scope of the claims set out below.

## Claims

1. A front end (20) for use in fluid communication with an inflatable body (1) of a blood pressure measuring device (10), the front end comprising:
i. a gas conduit (C) comprising a first portion (I) connectable to an inflatable body (1) and a second portion (II) connectable to a gas supply unit (9b);
ii. a pressure sensor (5) for measuring pressure in the first portion;
iii. a control valve (4) for controlling pressure in the inflatable body (1);
iv. an air chamber (4a) arranged in the second portion (II) of the gas conduit (C) for providing a buffer gas volume upstream the control valve (4) during inflation of the inflatable body (1), wherein the air chamber (4a) is arranged directly before the control valve (4) with respect to a direction of the stream towards the inflatable body (1); **characterised in that**
the frontend (2) is accommodated in an injection-molded plastic housing, the air chamber (4a) forming part of the housing.

2. A front end according to claim 1, wherein a bottom portion (21b) of the housing of the frontend (40) includes co-moulded cavities for the gas chamber (4a), for the control valve (4) and for the pressure sensor (5').

3. A front end according to claim 2, wherein a part of the first portion (I) of the gas conduit (C) traversing the inner volume of the frontend (40) is co-molded as well.

4. A front end according to any of the preceding claims, wherein the frontend (2) further comprises a gas connector (8) to the inflatable body (1), wherein the gas connector is connected by means of a hose.

5. A front end according to any of the preceding claims, wherein the housing comprises a cover portion (21a) and a bottom portion (21b).

6. A blood pressure measuring device (10) comprising:
- an inflatable body (1);
- a frontend (2) according to any of the preceding claims, in fluid communication with the inflatable body (1);
- a control system for operating the control valve (4);
- a system for measuring a signal representative of the blood
pressure arranged in the inflatable body (1), and wherein the inflatable body (1) comprises a cuff, preferably a finger cuff.

7. A device (10) according to claim 6, wherein the control system is arranged for analyzing a signal representative of pressure measured by the pressure sensor and for generating a control signal to the control valve (4) in response to said signal.

8. A device (10) according to any one of the claims 6-7, wherein the control system is arranged for analyzing a further signal from the system for measuring a signal representative of blood pressure and for generating a further control signal to the control valve (4) in response to said further signal.

9. A device (10) according to claim 8, wherein the control system is arranged to maintain a pressure level in the inflatable body (1) for enabling the system for measuring blood pressure to generate a stable signal.

10. A device (10) according to any one of the claims 6-9, wherein the system for measuring a signal representative of blood pressure comprises a photoplethysmograph.

11. A device (10) according to any one of the claims 6-10, wherein the device (10) further comprises a processor for collecting data representative of a blood pressure measurement.

12. A device (10) according to any one of the claims 6-11, comprising a plurality of differently sized inflatable bodies (1).

13. A device (10) according to claim 6, further comprising an identifying unit for identifying a size of an inflatable body being connected to the device.

14. A device (10) according to claim 13, further comprising a control program arranged to adapt a control algorithm of the control valve (4) in accordance with the size of the inflatable body and to cause the control system to carry-out the adapted control algorithm, the identifying unit providing input data to the control program.

15. A device (10) according to claim 13 or 14, wherein the control program is further arranged to adapt the control algorithm in accordance with parameters of the signal generated by the system for blood pressure measurement.

## Patentansprüche

1. Vorderes Ende (20) zur Verwendung in Fluidkommunikation mit einem aufblasbaren Körper (1) eines Blutdruckmessgeräts (10), wobei das vordere Ende Folgendes umfasst:
i. eine Gasleitung (C), umfassend einen ersten Abschnitt (I), anzuschließen an einen aufblasbaren Körper (1), und einen zweiten Abschnitt (II), anzuschließen an eine Gasversorgungsvorrichtung (9b);
ii. einen Drucksensor (5) zum Messen des Drucks in dem ersten Abschnitt;
iii. ein Steuerventil (4) zum Steuern des Drucks in dem aufblasbaren Körper (1);
iv. eine Luftkammer (4a), angeordnet im zweiten Abschnitt (II) der Gasleitung (C), um während des Aufblasens des aufblasbaren Körpers (1) stromaufwärts von dem Steuerventil (4) ein Puffergasvolumen bereitzustellen, wobei die Luftkammer (4a) unmittelbar vor dem Steuerventil (4) in Bezug auf eine Strömungsrichtung zum aufblasbaren Körper (1) hin angeordnet ist, **dadurch gekennzeichnet, dass**
das vordere Ende (2) in einem im Spritzgussverfahren hergestellten Kunststoffgehäuse untergebracht ist, wobei die Luftkammer (4a) einen Teil des Gehäuses bildet.

2. Vorderes Ende nach Anspruch 1, wobei ein unterer Abschnitt (21b) des Gehäuses des vorderen Endes (40) mitgeformte Hohlräume für die Gaskammer (4a), für das Steuerventil (4) und für den Drucksensor (5') aufweist.

3. Vorderes Ende nach Anspruch 2, wobei ein Teil des ersten Abschnitts (I) der Gasleitung (C), der durch das Innere des vorderen Endes (40) verläuft, ebenfalls mitgeformt ist.

4. Vorderes Ende nach einem der vorhergehenden Ansprüche, wobei das vordere Ende (2) ferner eine Gasverbindung (8) zm aufblasbaren Körper (1) umfasst, wobei die Gasverbindung mithilfe eines Schlauchs verbunden ist.

5. Vorderes Ende nach einem der vorhergehenden Ansprüche, wobei das Gehäuse ein Abdeckteil (21a) und Bodenteil (21b) umfasst.

6. Blutdruckmessgerät (10) umfassend:
- einen aufblasbaren Körper (1);
- ein vorderes Ende (2) nach einem der vorhergehenden Ansprüche, in Fluidkommunikation mit dem aufblasbaren Körper (1);
- ein Steuersystem zum Bedienen des Steuerventils (4);
- ein System zum Messen eines für den Blutdruck repräsentativen Signals, angeordnet in dem aufblasbaren Körper (1),
und wobei der aufblasbare Körper (1) eine Manschette, vorzugsweise eine Fingermanschette umfasst.

7. Gerät (10) nach Anspruch 6, wobei das Steuersystem geeignet ist zum Analysieren eines Signals, das repräsentativ für den von dem Drucksensor gemessenen Blutdruck ist, und zum Erzeugen eines Steuersignals zm Steuerventil (4) in Reaktion auf das Signal.

8. Gerät (10) nach einem der Ansprüche (6-7), wobei das Steuersystem geeignet ist zum Analysieren eines weiteren Signals von dem System zum Messen eines Signals, das repräsentativ für den Blutdruck ist, und zum Erzeugen eines weiteren Steuersignals zm Steuerventil (4) in Reaktion auf das Signal.

9. Gerät (10) nach Anspruch 8, wobei das Steuersystem geeignet ist, eine Blutdruckhöhe in dem aufblasbaren Körper (1) aufrechtzuerhalten, um es Blutdruckmesssystem zu ermöglichen, ein stabiles Signal zu erzeugen.

10. Gerät (10) nach einem der Ansprüche 6-9, wobei das System zum Messen eines für den Blutdruck repräsentativen Signals einen Photoplethysmograph umfasst.

11. Gerät (10) nach einem der Ansprüche 6-10, wobei das Gerät (10) ferner einen Prozessor zum Sammeln von Daten, die repräsentativ für eine Blutdruckmessung sind, umfasst.

12. Gerät (10) nach einem der Ansprüche 6-11, umfassend mehrere verschieden große aufblasbare Körper (1).

13. Gerät (10) nach Anspruch 6, ferner umfassend eine Identifikationseinheit zum Identifizieren einer Größe eines aufblasbaren Körpers, der mit dem Gerät verbunden ist.

14. Gerät (10) nach Anspruch 13, ferner umfassend ein Steuerprogramm, geeignet um einen Steueralgorithmus des Steuerventils (4) entsprechend der Größe des aufblasbaren Körpers anzupassen und das Steuersystem zu veranlassen, den angepassten Steueralgorithmus auszuführen, wobei die Identifikationseinheit Eingabedaten für das Steuerprogramm liefert.

15. Gerät (10) nach Anspruch 13 oder 14, wobei das Steuerprogramm ferner geeignet ist, um den Steueralgorithmus entsprechend den Parametern des von dem Blutdruckmesssystem erzeugten Signals anzupassen.

## Revendications

1. Embout avant (20) destiné à servir en communication fluidique avec un corps gonflable (1) d'un dispositif de mesure (10) de pression sanguine, l'embout avant comportant :
i. un conduit (C) de gaz comprenant un premier élément (I) raccordable à un corps gonflable (1) et un second élément (II) raccordable à une source (9b) de gaz ;
ii. un capteur (5) de pression pour mesurer la pression dans la première partie ;
iii. un régulateur (4) pour réguler la pression dans le corps gonflable (1) ;
iv. une chambre (4a) d'air disposée dans le second élément (II) du conduit (C) de gaz pour créer un volume tampon de gaz en amont du régulateur (4) pendant le gonflage du corps gonflable (1), la chambre (4a) d'air étant disposée juste avant le régulateur (4) par rapport à un sens d'écoulement du flux vers le corps gonflable (1) ; **caractérisé en ce que**
l'embout avant (2) est logé dans un boîtier en matière plastique moulé par injection, la chambre (4a) d'air faisant partie du boîtier.

2. Embout avant selon la revendication 1, dans lequel un élément formant fond (21b) du boîtier de l'embout avant (40) comprend des cavités formées par co-moulage pour la chambre (4a) d'air, pour le régulateur (4) et pour le capteur (5') de pression.

3. Embout avant selon la revendication 2, dans lequel une partie du premier élément (I) du conduit (C) de gaz traversant le volume intérieur de l'embout avant (40) est également formée par co-moulage.

4. Embout avant selon l'une quelconque des revendications précédentes, l'embout (2) comportant en outre un raccord (8) à gaz relié au corps gonflable (1), le raccord à gaz étant relié par l'intermédiaire d'un flexible.

5. Embout avant selon l'une quelconque des revendications précédentes, dans lequel le boîtier comprend un élément formant couvercle (21a) et un élément formant fond (21b).

6. Dispositif de mesure (10) de pression sanguine, comportant :
- un corps gonflable (1) ;
- un embout avant (2) selon l'une quelconque des revendications précédentes, en communication fluidique avec le corps gonflable (1) ;
- un système de commande pour actionner le régulateur (4) ;
- un système, disposé dans le corps gonflable (1),pour mesurer un signal représentatif de la pression sanguine,
et dans lequel le corps gonflable (1) comprend un brassard, de préférence un brassard digital.

7. Dispositif (10) selon la revendication 6, dans lequel le système de commande est conçu pour analyser un autre signal représentatif d'une pression mesurée par le capteur de pression et pour produire, en réponse audit autre signal, encore un autre signal de commande appliqué au régulateur (4).

8. Dispositif (10) selon l'une quelconque des revendications 6 et 7, dans lequel le système de commande est conçu pour analyser un autre signal émanant du système pour mesurer un signal représentatif d'une pression sanguine et pour produire, en réponse audit autre signal, encore un autre signal de commande appliqué au régulateur (4).

9. Dispositif (10) selon la revendication 8, dans lequel le système de commande est conçu pour maintenir un niveau de pression dans le corps gonflable (1) afin de permettre au système de mesurer une pression sanguine de manière à produire un signal stable.

10. Dispositif (10) selon l'une quelconque des revendications 6 à 9, dans lequel le système pour mesurer un signal représentatif d'une pression sanguine comprend un photopléthysmographe.

11. Dispositif (10) selon l'une quelconque des revendications 6 à 10, le dispositif (10) comportant en outre un processeur pour recueillir des données représentatives d'une mesure de pression sanguine.

12. Dispositif (10) selon l'une quelconque des revendications 6 à 11, comportant une pluralité de corps gonflables (1) de différentes tailles.

13. Dispositif (10) selon la revendication 6, comportant en outre un moyen d'identification pour identifier une taille d'un corps gonflable raccordé au dispositif.

14. Dispositif (10) selon la revendication 13, comportant en outre un programme de commande conçu pour adapter un algorithme de commande du régulateur (4) en fonction de la taille du corps gonflable et pour amener le système de commande à exécuter l'algorithme de commande adapté, le moyen d'identification fournissant des données d'entrée au programme de commande.

15. Dispositif (10) selon la revendication 13 ou 14, dans lequel le programme de commande est en outre conçu pour adapter l'algorithme de commande en fonction de paramètres du signal produit par le système pour la mesure d'une pression sanguine.
